# EUROPEAN PATENT SPECIFICATION

(11) **EP 2 394 604 B1**
(45) Date of publication and mention of the grant of the patent: **26.03.2014**
(21) Application number: 10709241.3
(22) Date of filing: 02.02.2010
(51) Int. Cl.: A61F 2/00

(54) **DYNAMIC CERCLAGE DEVICE FOR THE TREATMENT OF ANAL INCONTINENCE**
DYNAMISCHE CERCLAGE-VORRICHTUNG ZUR BEHANDLUNG VON STUHLINKONTINENZ
DISPOSITIF DE CERCLAGE DYNAMIQUE POUR LE TRAITEMENT DE L'INCONTINENCE ANALE

(30) Priority: 03.02.2009 ES 200900165 U
(43) Date of publication of application: 14.12.2011
(73) Proprietor: Devesa Mugica, José Manuel, 28109 Madrid (ES)
(72) Inventor: Devesa Mugica, José Manuel, 28109 Madrid (ES)
(74) Representative: Colò, Chiara
(86) International application number: PCT/ES2010/070055
(87) International publication number: WO 2010/089440

(56) References cited:
- EP-A2- 0 207 426
- US-A- 1 810 027
- US-A- 2 271 927
- US-A1- 2002 007 222

## Description

### Field of the Invention

The present invention relates to a device for the treatment of anal incontinence, to be used in the surgery field.

### Background Art

Anal incontinence consists of an involuntary escape of gases and feces. This disease results in major troubles which severely affect the personal, familiar, working, social life and the quality of life in general. Anal incontinence is estimated to affect 2.2% of the people, with figures up to 60% in geriatric residences, which means that, apart from the personal cost, the sanitary cost of this problem, including direct and indirect expenses, is very important.

Surgical and rehabilitation procedures are usually used for treating anal incontinence, however, whenever these ones fail, it is necessary to use different surgical techniques or operations. At present, all of these techniques feature a variable, however generally high, percentage of failures.

In those cases in which incontinence is caused by an anatomical or functional deficiency of the anal sphincter, the resistance to the coming out of the rectal content is insufficient, thus causing involuntary escape of gases and feces. In these quite numerous cases, one of the aims of the treatment consists of creating a pressure barrier around the anus, so as to keep it closed. Sometimes this can be achieved by operating on the sphincter itself, or by creating a substitutive sphincter through the use of another muscle of the body.

If the previous solutions are not possible or fail, then another technique used consists of implanting a ring made of some material or cerclage around the final section of the rectum and anus. The original anal cerclage technique was described by Thiersch in 1895, using a silver wire. Since then, a number of modifications were introduced with respect to the material used: silk, fascia, sinew, Teflon® tubes, nylon and polyester strips, Marlex® nylon meshes, and Silastic sheets. In general, even though the initial results obtained were frequently excellent, in the end these performances worsened in many cases, because of the biological, mechanical, and morphological characteristics of the device used. One of the last improvements of anal cerclage introduced is that called artificial anal sphincter (ACTICON, AMS)®, an hydraulic device formed of three components parts.

However, all devices presently used feature a high percentage of failures, which are due to the use of little appropriate materials, featuring a bad tolerance, or to an inappropriate physical configuration, resulting in a foreign body that erodes the tissues, by migrating from its original position and eroding the anal or skin structures.

Another cause for such a malfunctioning consists of the use of a rigid and inextensible ring. Such a configuration might prevent a complete rectal evacuation, thus creating a problem contrary to that to be corrected, and also prevent the access to the lower section of the intestine for clinical explorations, which are frequently necessary to make.

An ideal device for an undefined preservation of an anal cerclage must include the following characteristics:
1.- To be biotolerable and such as not to produce a local fibrous reaction, which would make difficult its extraction or replacement, if necessary.
2.- To be elastic, not rigid, to allow a better closing pressure and the possibility of relaxation for the evacuation function and the possible realization of transanal diagnostic studies, which are so frequent and necessary, including colonoscopy.
3.- To be radio-opaque, to be able to have an image of its status whenever so requested in the case of any disfunctions over time, which would allow to know about its effectiveness or spontaneous breakage, through a simple radiography of pelvis.
4.- Not to include metal elements that might interfere with or prevent the realization of image studies, including Magnetic Resonance.
5.- Not to have edges that might cause erosions of the skin, which would make it necessary to extract it, by preventing edges and thin sheets, whereby it should feature a blunt profile with a wide section everywhere.
6.- To be provided with a simple, effective and reversible closing system, should it be necessary to tighten or loose it more, depending on the functional result obtained.
7.- To be easy to implant, in accordance with the technical simplicity used with the previously described material, in order for it to be implantable by surgeons not highly specialized in anus-rectal surgery and consequently to be applicable to a much higher number of patients.
8.- To feature a low manufacturing cost, so as to allow its universalization, including countries having poor economical resources, and to also address patients featuring minor degrees of incontinence who, considering the high cost and possible complications of the mentioned alternative procedures, presently don't have any other possibility of improvement.

Document US 2002/0007222 discloses a device supporting a corporal organ configured in the form of a fastener, with a first longitudinal section having a number of openings in which a second longitudinal section with sawtooth edges inserts to form a band, encircling and supporting a corporal organ and possibly used, for example, as a band for the treatment of sphincter deficiencies, including the anal sphincter ones. Here, the first longitudinal section is realized in the form of a solid strip with straight edges, or in the form of a structure of multiple layers all around a central solid strip and, when the toothed longitudinal section inserts in the openings of the first longitudinal section to form the band that encircles the corporal organ, and, even in the case of being trimmed features a toothed termination that results in a potential risk of damages and lesions in the corporal structures close to the corporal organ supported.

The dynamic cerclage device for the treatment of anal incontinence that is the aim of this invention has been designed to solve the problems found with the present cerclage techniques.

This aim is achieved according to the present invention thanks to the characteristics indicated in claim 1. Other advantages of the present invention result from the characteristics indicated in the dependent claims. The starting point is a general disposition device in the form of a fastener comprising:
- a main body made of an elastomeric material arranged in such a way as to form a ring which rests around a portion of organ, in the form of a tube, for example a portion of the intestinal rectum; and
- a closure tongue configured as one piece as an extension of one of the ends of the main body, such a closure tongue having a free end shaped as an arrowhead and a plurality of sawteeth placed at equal distances all along it and whose tongue, upon inserting in a locking aperture located at an end of the main body, remains trapped, so that the aforementioned main body basically forms a closed ring;
which is characterized in accordance with the invention in that:
- the main body is shaped as an hollow section with at least two thick laminar walls that form their respective inner and outer faces of the ring, which join, without forming sharp edges, to their respective thinner laminar walls, which form the blunt edges of the ring;
- in the thick laminar walls there are a plurality of perforations equally spaced from each other, over lines extending longitudinally along said main body;
- close to the end opposite to the closure tongue, the main body comprises a number of protuberances shaped as mushroom-like closing buttons;
- the sawteeth of the closure tongue are shaped rounded-off/blunt and each of them is provided with a central hole for a tight-fitting, but dismountable, accommodation of the mushroom-shaped protuberances of the main body whenever this one closes to form a ring.

Since the main body consists of a hollow section with perforations, its longitudinal direction is intrinsically elastic, so as to also present an increased radial elasticity when closing around the corporal organ.

Thanks to the tube-like configuration of the main body, the end of the main body close to the closure tongue can be adapted to exactly insert through the opposite end of said main body whenever the cerclage device is closed to form a ring, so that the closure tongue can go through outwards via the locking aperture, while lying parallel to one of the thick portions of wall of said main body.

Moreover, in order to avoid risks of damages in the corporal structures that encircle the fastened organ, the sawteeth of the closure tongue are configured blunt, without any cutting portions, and the remaining section of the closure tongue can be fixed firmly leaning against the main body, thus avoiding any loose sections.

For a better understanding of the characteristics of this invention, a detailed description is given below, with reference to a number of drawings accompanying this description. Said drawings show:

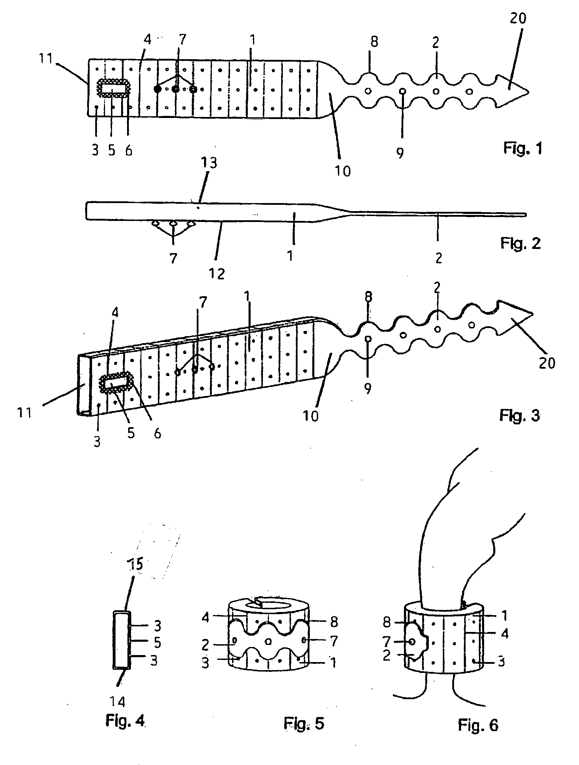

- Figure 1: a front view of the system here described.
- Figure 2: an upper view of the model shown in the previous figure.
- Figure 3: an isometric perspective view of the model. A section of the end.
- Figure 4: a section of the device in its component part corresponding to its closure system.
- Figure 5: an isometric perspective of the closed device in its final arrangement.
- Figure 6: a perspective view of the closed system applied around the final part of the rectum and anus.

With reference to the drawings, the device here envisaged comprises a main body 1. Said body is a hollow section or tube and makes up longitudinally a widened strip. This main body 1 comprises a closure tongue 2 at one of its ends, so as to form one piece.

The main body 1 that makes-up the actual cerclage ring is a hollow section featuring a rectangular or, alternatively, approximately elliptical transversal section and is shaped with a laminar wall with two thick portions of wall 12, 13, which make-up their respective inner and outer faces of the ring and two thinner portions of wall 14, 15, which join together without forming sharp edges to form the blunt edges of the ring. The main body 1 consists of an elastomeric-base material, for example silicone, preferably radio-opaque, and its thick portions of wall 12, 13 include perforations 3 in the form of millimetric pinholes arranged on lines, for example three, parallel to the edges of the ring and spaced from each other, for example by 1 cm, which results in an elasticity adequate to its aperture to make rectal evacuation easier, while also retaining an adequate closure resistance. The upper and lower edges are blunt. The main body assembly 1 features an approximate height of, for example, 1.5 cm, which is the width of the inner sphincter and the high physiological pressure area; and a length of, for example, 10 cm, so as to cover the maximum possible length, being it trimmable after being definitively adjusted to prevent any unnecessary material which might cause erosions of the skin and of the other corporal structures nearby. The outer part of the main body 1 comprises a sequence of marks 4, having the shape of etched or printed vertical lines, spaced from each other by, for example, 1 cm, and sequentially numbered, which allows to know the length of the ring implanted after closing it. For instance, at a distance of say 8 millimeters from its final end, i.e. in correspondence with the remote end of the closure tongue 2, on its outer face, there is a, for example, 1.4 cm long aperture 5, through which it is possible to insert the end of the closure tongue 2, as necessary to adjust the closing pressure. The perimeter 6 of the locking aperture 5 is reinforced to prevent the closure tongue from displacing backwards and consequently the ring from opening after being implanted. Close to the end opposite to the closure tongue and just after said locking aperture 5, on the outer face of the ring, i.e. in the thick portion of wall 12 and on its central line, there are a number, for example three, of button-like mushroom-shaped protuberances 7 used to insert, as better described below, in openings 9 correspondingly provided at the middle of the blunt teeth 8 of the closure tongue 2, after the cerclage device is closed to form the ring, this way providing an additional security closure, which is also used in order for the part of the tongue in excess to remain closely adherent to the ring and prevent the potential erosion of the skin in areas close to the cerclage device after its implantation.

As already mentioned, the closure tongue 2 is nothing but the extension of an end 10 of the main body 1 and features a compact section, with a reinforcement of its consistency exactly as the frame of the opening of the ring through which it inserts, to prevent its displacement backwards; its shape is wavy or saw-like, with a number, for example four, of blunt teeth 8 each, say, 1.3 cm long, which allows them to enter the aperture 5 of the main body 1 when at its place. It can be noted that this end 10 of the main body 1 close to the closure tongue features a configuration suitable for being exactly introduced through the opposite end 11 of said main body, when the cerclage device closes to form a ring, this way the closure tongue 2 can be made pass through outwards via the locking aperture 5, while lying parallel to one of the thick portions of wall 12 of said main body 1. At the middle of every blunt tooth 8 there is a hole or pinhole 9 whose width is less than the maximum diameter of the head of the mushroom-shaped buttons that make-up the protuberances 7 of the main body 1, so that, once inserted, the buttons cannot come off and assure an additional closure of the device and, as already mentioned before, make the section of the closure tongue 2 in excess remain firmly adherent to the ring, thus preventing a potential erosion of the skin.

Said system allows to re-adjust the closing pressure if, after the initial implantation of the ring, it is found to be too much tightened, which makes the evacuation of the rectal content difficult, or too much loose, which allows discharges thereof.

As already mentioned, the main body 1 lies parallel to the rectum, and is implanted at the height of the defective anal sphincter.

## Claims

1. Dynamic cerclage device for the treatment of anal incontinence, generally arranged in the form of a fastener comprising:
- a main body (1) made of an elastomeric material arranged to form a ring which rests around a portion of organ in the form of a tube, like a portion of the intestinal rectum; and
- a closure tongue (2) configured in one piece as an extension of one of the ends (10) of the main body (1), such a closure tongue having a free end (20) shaped as an arrowhead and a plurality of teeth (8) located all along it and whose tongue, upon inserting through a locking aperture (5) located at an opposite end (11) of the main body (1), remains trapped so that the aforementioned main body basically forms a closed ring;
**characterized in that**:
- the main body (1) is configured as a hollow section or tube, with a laminar side wall having at least two thick portions of wall (12, 13) that form their respective inner and outer faces of the ring, which join, without forming sharp edges, to their respective thinner portions of wall (14, 13) that form the blunt edges of the ring upon closing the cerclage device;
- the end (10) of the main body (1) close to the closure tongue (2) is suitable for being exactly inserted through the opposite end (11) of said main body when the cerclage device is closed to form a ring, so that the closure tongue (2) can be passed through outwards via the locking aperture (5), while lying parallel to one of the thick portion of wall (12) of said main body (1);
- in said thick portions of wall (12, 13) there are a plurality of perforations (3) equally spaced from each other over lines extending in the longitudinal direction of said main body;
- close to the end (11) opposite to the closure tongue (2), the main body (1) is provided with protuberances (7) shaped as mushroom-like closing buttons extending from the locking aperture (5) in the direction towards the closure tongue (2);
- the teeth (8) of the closure tongue are configured rounded-off or blunt to prevent cutting edges and a hole (9) is configured in each of said blunt teeth for an exact, but dismountable, accommodation of the mushroom-shaped protuberances (7) of the main body (1) whenever this one is closed to form a ring.

2. Dynamic cerclage device for the treatment of anal incontinence according to claim 1, **characterized in that** this one is made of silicone, preferably a radio-opaque silicone.

3. Dynamic cerclage device for the treatment of anal incontinence according to at least one of the previous claims, **characterized in that** the locking aperture (5) of the main body (1) features a reinforced perimeter (6) to increase its mechanical strength.

4. Dynamic cerclage device for the treatment of anal incontinence according to at least one of the previous claims, **characterized in that** the thick portions of wall (12, 13) of the main body (1) feature a width suitable for reproducing the width of the inner sphincter and the high physiological pressure area.

5. Dynamic cerclage device for the treatment of anal incontinence according to claim 4, **characterized in that** the thick portion of wall (12, 13) of the main body (1) feature a width of approximately 1.5 cm.

6. Dynamic cerclage device for the treatment of anal incontinence according to the previous claims, **characterized in that** the length of the main body (1) is selected to cover the max possible length, being trimmable after its definitive adjustment to prevent unnecessary materials that might cause erosions of the skin.

7. Dynamic cerclage device for the treatment of anal incontinence according to claim 6, **characterized in that** the length of the main body (1) is approximately 10 cm.

8. Dynamic cerclage device for the treatment of anal incontinence according to the previous claims, **characterized in that** at least the outer face of the main body (1), i.e. one of the thick portions of wall (12), is provided with a sequence of marks (4) in the form of separated etched or printed vertical lines and sequentially numbered, which allows to know the length of the implanted ring after closing it, and allows to know the reference of the closing length, should it be necessary to replace it in the future with another similar device, or to re-adjust it at higher or lower levels.

9. Dynamic cerclage device for the treatment of anal incontinence according to claim 8, **characterized in that** the marks (4) are spaced by 1 cm from each other.

## Patentansprüche

1. Dynamische Cerclage-Vorrichtung zur Behandlung von Stuhlinkontinenz, die im Allgemeinen in Form eines Befestigers angeordnet ist, umfassend:
- einen Hauptkörper (1), der aus einem Elastomermaterial hergestellt ist, das so angeordnet ist, dass es einen Ring formt, der um einen Organabschnitt herum in Form eines Schlauchs angeordnet ist, wie einen Abschnitt des Mastdarms; und
- eine Verschlusszunge (2), die einstückig als eine Verlängerung eines der Enden (10) des Hauptkörpers (1) konfiguriert ist, wobei solche eine Verschlusszunge ein freies Ende (20), das wie eine Pfeilspitze gestaltet ist, und eine Vielzahl von Zähnen (8) die alle an ihr entlang liegen, aufweist, und deren Zunge beim Einführen durch eine Feststellöffnung (5), die an einem entgegengesetzten Ende (11) des Hauptkörpers (1) liegt, festsitzt, sodass der zuvor genannte Hauptkörper im Prinzip einen geschlossenen Ring formt;
**dadurch gekennzeichnet, dass**:
- der Hauptkörper (1) als ein Hohlteil oder Schlauch konfiguriert ist, wobei eine laminare Seitenwand mindestens zwei dicke Wandabschnitte (12, 13) aufweist, die ihre jeweilige Ringinnen- und-außenfläche formen, die sich ohne das Formen von scharfen Kanten mit ihren jeweiligen dünneren Wandabschnitten (14, 15) zusammenschließen, welche die stumpfen Kanten des Rings beim Schließen der Cerclage-Vorrichtung formen;
- das Ende (10) des Hauptkörpers (1) in der Nähe der Verschlusszunge (2) dazu geeignet ist, exakt durch das entgegengesetzte Ende (11) des Hauptkörpers hindurch eingeführt zu werden, wenn die Cerclage-Vorrichtung geschlossen ist, um einen Ring zu formen, sodass die Verschlusszunge (2) über die Feststellöffnung (5) nach außen durchgeführt werden kann, während sie parallel zu einem der dicken Wandabschnitte (12) des Hauptkörpers (1) liegt;
- in den dicken Wandabschnitten (12, 13) eine Vielzahl von Perforationen (3) vorhanden ist, die gleichmäßig über Linien beabstandet sind, die sich in der Längsrichtung des Hauptkörpers erstrecken;
- in der Nähe des Endes (11) entgegengesetzt zur Verschlusszunge (2) der Hauptkörper (1) mit Vorsprüngen (7) ausgestattet ist, die als pilzähnliche Verschlussknöpfe gestaltet sind, die sich von der Feststellöffnung (5) in Richtung der Verschlusszunge (2) erstrecken;
- die Zähne (8) der Verschlusszunge abgerundet oder stumpf konfiguriert sind, um Schnittkanten zu verhindern, und in jedem der stumpfen Zähne ein Loch (9) für eine exakte, aber zerlegbare Unterbringung der pilzähnlichen Vorsprünge (7) des Hauptkörpers (1) konfiguriert ist, und zwar immer dann, wenn dieser geschlossen ist, um einen Ring zu formen.

2. Dynamische Cerclage-Vorrichtung für die Behandlung von Stuhlinkontinenz nach Anspruch 1, **dadurch gekennzeichnet, dass** diese aus Silikon, vorzugsweise aus einem röntgensichtbaren Silikon, hergestellt ist.

3. Dynamische Cerclage-Vorrichtung zur Behandlung von Stuhlinkontinenz nach mindestens einem der vorangehenden Ansprüche, **dadurch gekennzeichnet, dass** die Feststellöffnung (5) des Hauptkörpers (1) über einen verstärkten Perimeter (6) verfügt, um ihre mechanische Festigkeit zu vergrößern.

4. Dynamische Cerclage-Vorrichtung zur Behandlung von Stuhlinkontinenz nach mindestens einem der vorangehenden Ansprüche, **dadurch gekennzeichnet, dass** die dicken Wandabschnitte (12, 13) des Hauptkörpers (1) über eine Breite verfügen, die dazu geeignet ist, die Breite des inneren Schließmuskels und den Bereich mit hohem physiologischen Druck nachzubilden.

5. Dynamische Cerclage-Vorrichtung zur Behandlung von Stuhlinkontinenz nach Anspruch 4, **dadurch gekennzeichnet, dass** der dicke Wandabschnitt (12, 13) des Hauptkörpers (1) über eine Breite von ca. 1,5 cm verfügt.

6. Dynamische Cerclage-Vorrichtung zur Behandlung von Stuhlinkontinenz nach den vorangehenden Ansprüchen, **dadurch gekennzeichnet, dass** die Länge des Hauptkörpers (1) zum Bedecken der maximal möglichen Länge ausgewählt wird, wobei sie nach ihrer definitiven Anpassung kürzbar ist, um unnötiges Material zu vermeiden, das Erosionen der Haut verursachen könnte.

7. Dynamische Cerclage-Vorrichtung zur Behandlung von Stuhlinkontinenz nach Anspruch 6, **dadurch gekennzeichnet, dass** die Länge des Hauptkörpers (1) ca. 10 cm beträgt.

8. Dynamische Cerclage-Vorrichtung zur Behandlung von Stuhlinkontinenz nach den vorangehenden Ansprüchen, **dadurch gekennzeichnet, dass** zumindest die Außenfläche des Hauptkörpers (1), d. h. einer der dicken Wandabschnitte (12), mit einer Sequenz von Markierungen (4) in Form von einzelnen geätzten oder aufgedruckten vertikalen Linien, die sequentiell nummeriert sind, ausgestattet ist, wodurch ermöglicht wird, die Länge des implantierten Rings, nachdem er verschlossen ist, zu erkennen, und den Bezug auf die Verschlusslänge zu erkennen, falls es notwendig sein sollte, ihn in der Zukunft mit einer anderen ähnlichen Vorrichtung zu ersetzen oder ihn an höheren oder tieferen Stellen neu anzupassen.

9. Dynamische Cerclage-Vorrichtung zur Behandlung von Stuhlinkontinenz nach Anspruch 8, **dadurch gekennzeichnet, dass** die Markierungen (4) um 1 cm voneinander beabstandet sind.

## Revendications

1. Dispositif de cerclage dynamique pour le traitement de l'incontinence anale, se présentant généralement sous la forme d'un élément de fixation comprenant :
- un corps principal (1) fabriqué dans une matière élastomérique disposé de manière à former un anneau reposant autour d'une partie d'organe en forme de tube, comme une partie du rectum de l'intestin ; et
- une languette de fermeture (2) configurée en une seule pièce comme une extension d'une des extrémités (10) du corps principal (1), telle languette de fermeture ayant une extrémité libre (20) ayant la forme d'une pointe de flèche et une pluralité de dents (8) situées tout le long de celle-ci et laquelle languette, lors de l'insertion à travers une ouverture de verrouillage (5) située à une extrémité opposée (11) du corps principal (1), reste bloquée de sorte que le corps principal susmentionné a plus ou moins la forme d'un anneau fermé ;
**caractérisé en ce que** :
- le corps principal (1) est configuré comme une section creuse ou tube, avec une paroi latérale laminaire ayant au moins deux parties épaisses de paroi (12, 13) formant leurs faces interne et externe respectives de l'anneau, qui joignent, sans former d'arêtes vives, leurs parties plus fines respectives de paroi (14, 15) formant les arêtes émoussées de l'anneau lors de la fermeture du dispositif de cerclage ;
- l'extrémité (10) du corps principal (1) proche de la languette de fermeture (2) est adaptée pour être exactement insérée à travers l'extrémité opposée (11) dudit corps principal lorsque le dispositif de cerclage est fermé pour former un anneau, de sorte que la languette de fermeture (2) peut être passée vers l'extérieur par le biais de l'ouverture de verrouillage (5), alors qu'elle est parallèle à l'une des parties épaisses de paroi (12) dudit corps principal (1) ;
- dans lesdites parties épaisses de paroi (12, 13) se trouve une pluralité de perforations (3) équidistantes les unes des autres au-dessus de lignes se développant dans la direction longitudinale dudit corps principal ;
- près de l'extrémité (11) à l'opposé de la languette de fermeture (2), le corps principal (1) est pourvu de saillies (7) ayant la forme de boutons de fermeture en forme de champignon se développant à partir de l'ouverture de verrouillage (5) dans la direction de la languette de fermeture (2) ;
- les dents (8) de la languette de fermeture sont configurées arrondies ou émoussées pour empêcher toute pointe coupante et un orifice (9) est configuré dans chacune desdites dents émoussées pour parfaitement loger, tout en étant démontable, les saillies en forme de champignon (7) du corps principal (1) chaque fois que celui-ci est fermé pour former un anneau.

2. Dispositif de cerclage dynamique pour le traitement de l'incontinence anale selon la revendication 1, **caractérisé en ce que** celui-ci est en silicone, de préférence en silicone radio-opaque.

3. Dispositif de cerclage dynamique pour le traitement de l'incontinence anale selon au moins une des revendications précédentes, **caractérisé en ce que** l'ouverture de verrouillage (5) du corps principal (1) présente un périmètre renforcé (6) pour augmenter sa résistance mécanique.

4. Dispositif de cerclage dynamique pour le traitement de l'incontinence anale selon au moins une des revendications précédentes, **caractérisé en ce que** les parties épaisses de paroi (12, 13) du corps principal (1) présentent une largeur adaptée pour reproduire celle du sphincter interne ainsi que la zone de pression élevée physiologique.

5. Dispositif de cerclage dynamique pour le traitement de l'incontinence anale selon la revendication 4, **caractérisé en ce que** la partie épaisse de paroi (12, 13) du corps principal (1) présente une largeur d'environ 1,5 cm.

6. Dispositif de cerclage dynamique pour le traitement de l'incontinence anale selon les revendications précédentes, **caractérisé en ce que** la longueur du corps principal (1) est choisie pour couvrir la longueur maximale possible, étant coupable après son réglage définitif pour empêcher que des matières inutiles ne provoquent des érosions de la peau.

7. Dispositif de cerclage dynamique pour le traitement de l'incontinence anale selon la revendication 6, **caractérisé en ce que** la longueur du corps principal (1) est d'environ 10 cm.

8. Dispositif de cerclage dynamique pour le traitement de l'incontinence anale selon les revendications précédentes, **caractérisé en ce qu'**au moins la face externe du corps principal (1), c'est-à-dire une des parties épaisses de paroi (12), est pourvue d'une séquence de marquages (4) sous forme de lignes verticales gravées ou imprimées séparées et numérotées séquentiellement, qui permet de connaître la longueur de l'anneau implanté après sa fermeture, et permet de connaître la référence de la longueur de fermeture, dans le cas où il serait nécessaire de le remplacer à postériori par un dispositif similaire, ou de le réajuster à des niveaux supérieurs ou inférieurs.

9. Dispositif de cerclage dynamique pour le traitement de l'incontinence anale selon la revendication 8, **caractérisé en ce que** les marquages (4) sont espacés les uns des autres d'1 cm.
